# EUROPEAN PATENT SPECIFICATION

(11) **EP 2 470 249 B1**
(45) Date of publication and mention of the grant of the patent: **08.08.2018**
(21) Application number: 10807469.1
(22) Date of filing: 11.08.2010
(51) Int. Cl.: A61M 25/00, A61M 25/04, A61M 25/02

(54) **SUPRAPUBIC URETHRAL CATHETERS**
SUPRAPUBISCHE HARNRÖHRENKATHETER
CATHÉTERS

(30) Priority: 24.08.2009 GB 0914766
(43) Date of publication of application: 04.07.2012
(73) Proprietor: Alternative Urological Catheter Systems Limited, Bristol BS6 6LT (GB)
(72) Inventor: FENELEY, Roger, Charles, Leslie, Bristol BS8 4BW (GB)
(74) Representative: Addiss, John William
(86) International application number: PCT/GB2010/001523
(87) International publication number: WO 2011/023929

(56) References cited:
- WO-A1-98/33545
- WO-A2-95/10316
- CA-A1- 2 490 966
- GB-A- 2 343 847
- US-E- R E35 849

## Description

### Background of the Invention

### Field of Invention

The present invention relates to catheters for drainage of the urinary bladder. In particular, the invention relates to suprapubic urethral catheters and catheter systems.

### Summary of the Prior Art

Urinary catheterisation introduces major medical, social and economic issues. The catheter is a medical device used to drain urine from the bladder but it also provides a conduit for bacteria to enter the body. One out of 4 hospitalized patients receives an indwelling catheter [1]. Catheter-associated urinary tract infections present the most common nosocomial infections, developing at an estimated rate of 5% per day and increasing the risk of bacteraemia and mortality [2]. Financial estimates in 2000 suggested that a symptomatic urinary tract infection raises the cost of care by around £420 and bacteraemia to at least £1750 by lengthening the stay in hospital and antibiotic therapy [3]. In developing countries the rates of healthcare-associated infections are 3 to 5 times higher than international standards [4] The self-retaining urinary catheter introduced by Dr Foley in 1937 has been the standard product in routine use for over 70 years [5]. The catheter is retained in the bladder by means of an inflatable balloon but this prevents the bladder from emptying completely, leaving residual infected urine. Long-term urinary catheterisation (LTC) provides a well-established method of management, draining urine from the bladder into a urine collection bag. LTC carries a high risk of complications; catheter-associated complications occur in over 70% of patients, the majority resulting from the rapid bacterial invasion of the catheter [6]. Urinary tract infection can lead to life threatening septicaemia but the common problems of encrustation and blockage of the catheter, urinary leakages and discomfort from the catheter have an immense impact not only on an individual's quality of life but on the costs to healthcare services [7]. The prevalence of LTC increases with age and although considered a last resort, many thousands of people rely on this method of urinary collection because no acceptable alternative is available [8] .

A Foley-type catheter is usually positioned in a patient by passing it up through the urethra. Such a catheter is known as a urethral catheter. However, Foley-type catheters may alternatively be inserted into the bladder through the abdominal wall just above the pubic bone i.e. along a suprapubic track. The latter is known as a suprapubic catheter.

Suprapubic urethral catheters are also known in the art. For example, see GB2343847A (also by the present inventor). A suprapubic urethral catheter is positioned in a patient via a suprapubic track and enables either suprapubic or urethral drainage of a urinary bladder. A suprapubic urethral catheter generally consists of a tube having, sequentially, a suprapubic section that will be located suprapubically in a patient, a bladder section that will be located within the bladder of the patient, and a urethral section that will be located within the urethra of the patient.

WO98/33545A1 discloses a suprapubic drainage catheter for draining the bladder and maintaining the patency of the urethra of a patient following a radical prostatectomy. The catheter includes an elongated member having a drainage passage extending longitudinally therein and a plurality of drainage ports in a drainage portion thereof for providing external access for the drainage passage in the bladder of the patient. First and second retention balloons are connected with the elongated member and positioned distally and proximally of the drainage ports, respectively. The elongated member also includes first and second inflation passages for individual and independent inflation and deflation of the retention balloons. One-way connector valves are positioned at the proximal end of the catheter for controlling inflation and deflation of the balloons via the inflation passages. The elongated member also includes a proximal portion that extends through the percutaneous access site for connection of a urine collection bag to the drainage passage. USRE35849E discloses an indwelling stent which aims to facilitate the drainage of fluids through a duct within the body, and which aims to be easily removed when needed. The disclosed stent is a double wing stent and includes multiple flaps at each end for anchoring and has a continuously uninterrupted drainage tube therebetween. Constructed at the outflow end of the stent is a wick, which is made of an extended arcical section of the drainage tube of the stent and serves to enhance drainage by providing a "wicking" effect for fluid as it exits the stent's outflow end, while inhibiting reflux by allowing for the sphincter at the exit of the obstructed duct to function in a normal manner. The wick further serves to facilitate removal by providing a portion which is easily graspable by an instrument inserted into the body for retention and removal.

WO95/10316A2 discloses a suprapubic catheter that comprises a drainage tube having a drainage end for location outside the patient's body. The drainage end has an enlarged head portion on which a urine collection bag may be removably mounted. The catheter has a disk which lies against the patient's skin. A breathable dressing is used to secure the disk in position and thus retain the catheter on the patient's body.

### Summary of the Invention

The present invention aims to overcome at least some of the aforementioned deficiencies in conventional self-retaining Foley-type catheters.

The present invention has been developed particularly for those patients who are experiencing complications with the conventional self-retaining Foley-type catheter and urine collection bag. Recurrent blocking of the catheter and bypassing of urine around the catheter are common problems associated with long-term urinary catheterisation. In addition the presentdescription describes a versatile method of urinary drainage and collectionthat may be indicated in patients presenting specific needs for urine collection. A system that mimics the cyclical filling and emptying of the bladder has been advocated to reduce the risk of urinary infections and the incidence of catheter blockages [9]; the present invention has been designed to provide this.

According to an aspect of the present invention, there is provided a urine collection system according to claim 1.

If pressure in the bladder rises above a threshold pressure, urine flows upstream and enters the urine collection container and then returns to the bladder when the pressure in the bladder falls below the threshold. For example, if pressure in the bladder rises as a result of a contraction of the bladder (detrusor) muscle, urine enters the urine collection container and then returns to the bladder as the bladder muscle relaxes.

Many patients requiring long-term catheterization suffer from a neurological disease or injury which accounts for their loss of bladder control. Under these conditions, the bladder can become overactive with inappropriate uncontrolled contractions both of the bladder and the sphincter closing the urethral passage during filling. This is termed detrusor sphincter dysynergia. These contractions cause the pressure to rise in the bladder which in turn creates back-pressure on the kidney(s) with the risk of serious damage to their function. The objective of the upstream urine collection container is to obviate the pressure rise by providing a low pressure urinary reservoir.

Preferably, the urine collection container is attachable to the abdomen of a patient, more preferably via a waistband. Preferably the urine collection container is a pouch, most preferably it is an abdominal urine collection pouch.

The urine collection container will preferably hold a volume of e.g. up to 500 ml of urine. The urine collection container may also act as a portal for drug administration.

A Luer fitting may be included at the top of the urine collection container so that the container and the catheter can be irrigated if necessary to clear any accumulated debris. The system optionally includes provision for irrigating the system. For example, normal saline can be flushed through the system.

The urine collection system may also optionally be as specified in any one of claims 5 to 19.

The present invention also provides a kit of parts according to claim 20, which may also optionally be as specified in any one of claims 21 or 22.

### Brief Description the Drawings

Embodiments of the present invention will now be described, by way of example, with reference to the accompanying diagrammatic drawings, in which:
Figure 1 shows a part cut away view of a catheter that can be used in an embodiment of the present invention.
Figure 2 shows a part cut away view of a catheter that can be used in an embodiment of the present invention
Figure 3 shows an abdominal stop that can be used in an embodiment of the present invention.
Figures 4a to 4e show a non-return cuff that can be used in embodiment of the present invention. Figure 4a is a 3-D view; Figure 4b is a longitudinal cross-sectional view; Figure 4c is an enlarged view of the area "A" circled in Figure 4b; Figure 4d is a side view; and Figure 4e is an end view.
Figures 5a and 5b show one embodiment of the present invention.

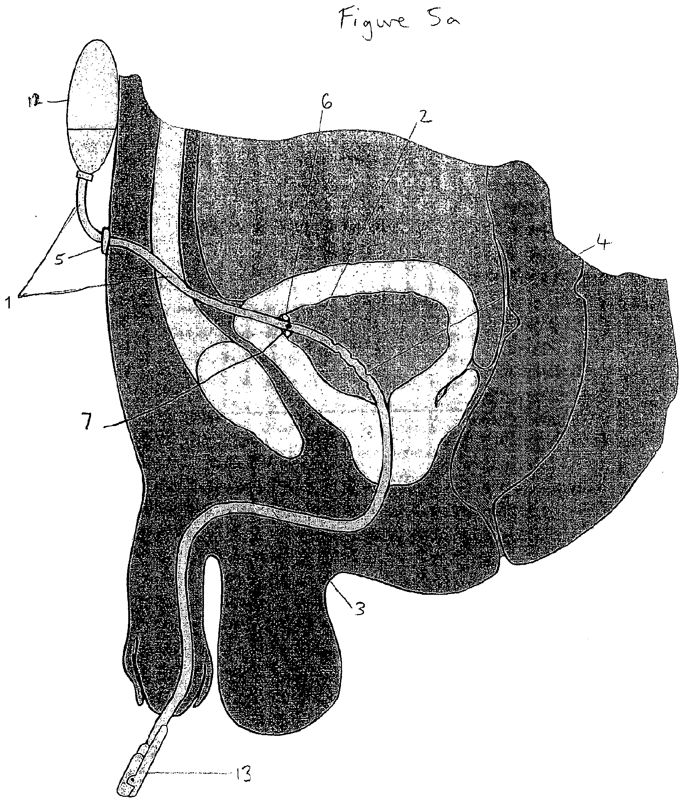

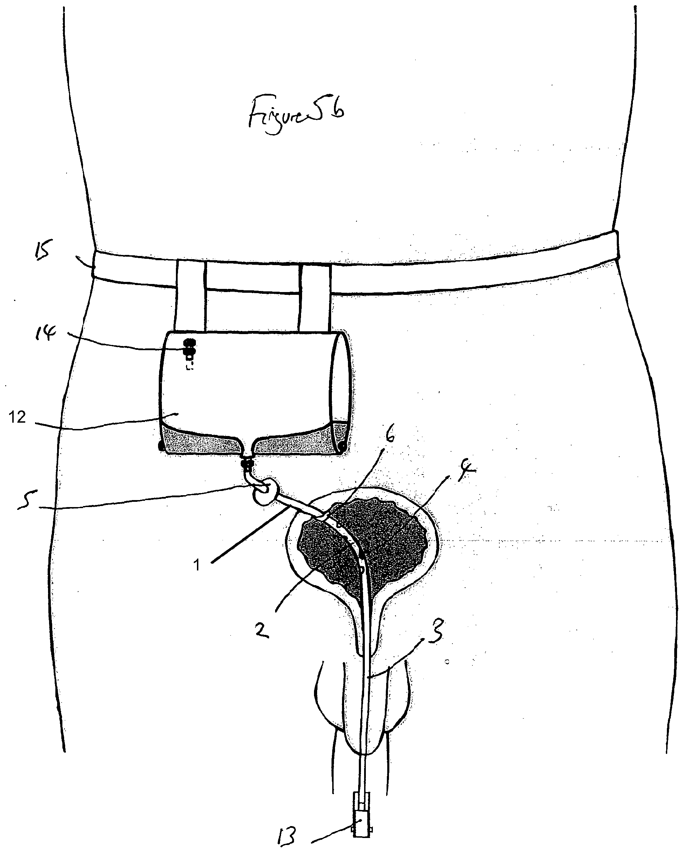

The embodiment shown consists of a catheter with a non-return cuff and the catheter is attached to an abdominal collection pouch. The Applicant has named this embodiment the Alternative Catheter System (ACS). Figure 5a shows a sagittal view of this embodiment in-situ in a male patient. Figure 5b shows a frontal view of this embodiment in-situ in a male patient.

### Detailed Description

The following detailed description of some embodiments of the invention should be read with reference to the drawings, wherein like reference numerals indicate like elements throughout the several views.

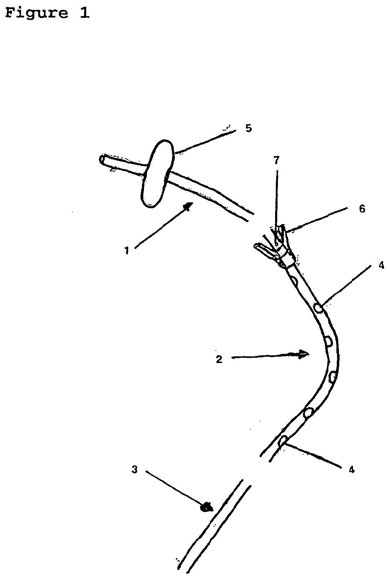

Referring to Figure 1, there is illustrated a part cut away view of a suprapubic urethral catheter that can be used in an embodiment of the present invention.

The catheter includes a flexible and hollow tube which may be described in three sections, namely a suprapubic section 1, a bladder section 2 and a urethral section 3. The tubing may be continuous, or there may be two or three sections of tubing joined together.

The suprapubic section 1 passes through an abdominal stop 5 (also referred to as an abdominal flange) which can be adjusted by sliding it along the catheter before the catheter enters the abdominal wall. The upper end of this section can be cut to a suitable length for the patient and can be fitted to an abdominal urine collection pouch or a drainage funnel or connector (not shown).

The bladder section 2 comes in various lengths depending on the size of the bladder. At its suprapubic or upper end it is fitted with a non-return cuff 6 that prevents the catheter from extruding retrogradely from the bladder and at its lower end, a radio-opaque, ultrasound or other marker (not shown in Figure 1) denotes its junction with the urethral section. A plurality of drainage eyeholes 4 are placed along the whole length of this section which enables the bladder to be drained completely of urine towards the urethral or lower end assisted by gravity or if this is closed through the upper end into a urine collection pouch (not shown in Figure 1).

The urethral section 3 is a tube of thinner wall thickness which can be cut to length appropriate to the individual patient and fitted into a drainage funnel or connector (not shown in Figure 1). A standard urethral valve (not shown in Figure 1) which can be opened or closed can be inserted into the drainage funnel to store in or empty urine from the bladder. A urine collection bag can be fitted to the catheter valve at the urethral end if necessary.

When located within a patient, the suprapubic end of the suprapubic section 1 of the catheter first passes through the abdominal stop 5, which grips and retains the catheter in position on the abdominal wall of the user, and then through the abdominal wall along a suprapubic track to join the bladder section 2 which is positioned in the bladder to drain urine. The bladder section leads on to the urethral section 3 which is positioned in the urethra. The location of the catheter within a patient can be seen from Figures 5a and 5b.

Referring to Figure 1 again, the bladder section 2 with the plurality of drainage eyeholes 4 includes a non-return cuff 6 at its upper end which prevents the catheter from extruding retrogradely out of the suprapubic track in the abdominal wall. Small lateral irrigation eyeholes 7 are placed in the catheter tubing of the bladder section positioned within the projecting portion of the cuff to wash out any accumulated debris.

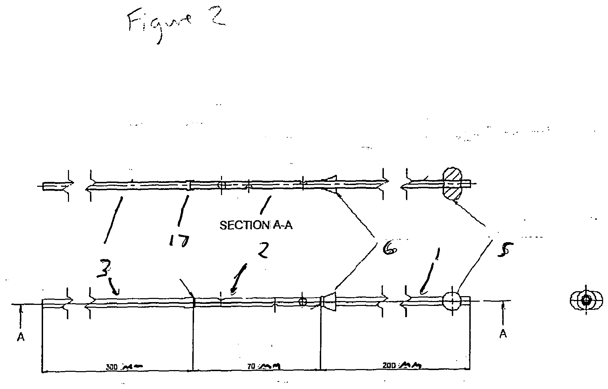

Referring to Figure 2, there is illustrated a catheter that can be used in an embodiment of the present invention. A non-return cuff 6 of a frusto-conical shape, and an abdominal stop 5 are shown attached to the catheter. Also shown is a radio-opaque or ultrasound marker 17 at the junction between the bladder section 2 and the urethral section 3.

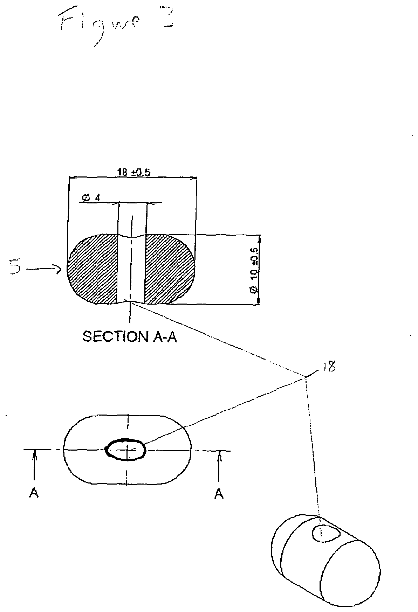

Referring to Figure 3, there is illustrated an abdominal stop 5 that can be used in an embodiment of the present invention. As can be seen, there is an oval channel 18 through the abdominal stop for receiving and gripping the suprapubic section of the catheter.

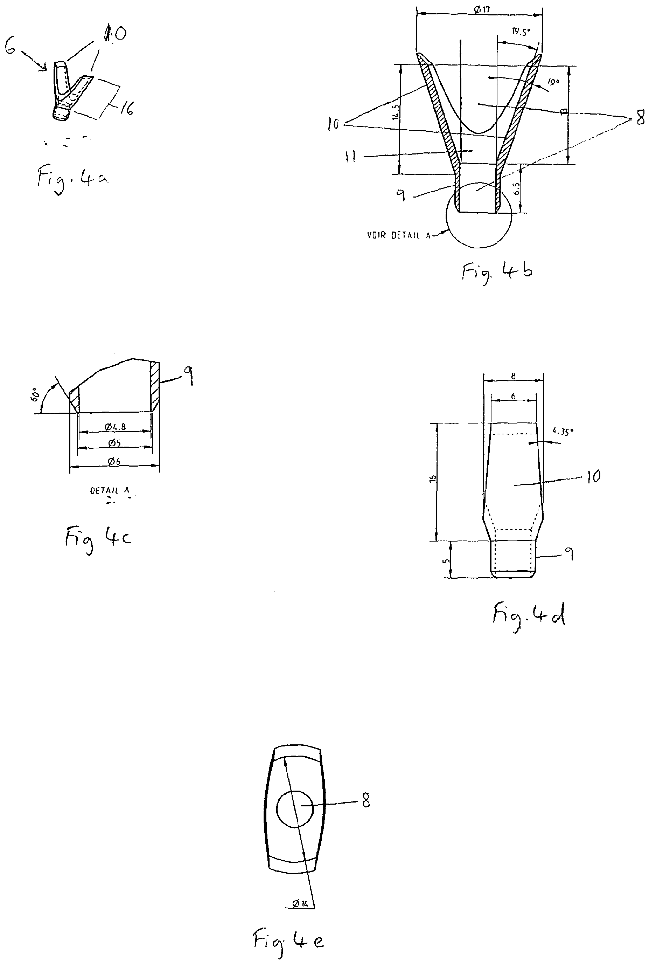

Referring to Figures 4a to 4e there is illustrated a non-return cuff 6 that can be used in an embodiment of the present invention. Figure 4a shows a non-return cuff having a projection portion 16 comprising two inclined wings 10. In other words the projection portion is substantially V-shaped. Figure 4b shows a longitudinal cross-section of the same embodiment of the non-return cuff. As can be seen from Figure 4b, the cuff has a channel 8 for receiving a catheter. The channel 8 extends throughout the centre of the cuff and between the two inclined wings 10. In other words the two inclined wings 10 are positioned on opposing sides of the channel 8. The inclined wings are joined by a resiliently deformable element 11 which biases the wings apart from each other. As can be seen from Figure 4b, at the end of the wings, where the distance between the wings is smallest, a sleeve 9 is formed around part of the channel 8. The ends of the sleeve 9, furthest from the wings, taper towards the channel. This is for ease of insertion of the cuff into a bladder along a suprapubic track defined by a catheter held within the channel of the cuff. This tapering can be most clearly seen from Figure 4c which provides an enlarged view of the area "A" circled in Figure 4b.

The structural configuration of the cuff is designed to balance the need for flexibility to allow ease of insertion with strength and stiffness to enable its non-return function. In the example shown in Figure 4d, the wings 10 have a distorted elongated polygonal shape, in which the widest part of the elongated polygon is near the end of the wings being closest to the sleeve, as shown in Figure 4d. This shape provides the arms with strength to resist deformation away from the channel whilst at the same time minimising any enlargement of the suprapubic track and the aperture into the bladder defined by the catheter on insertion.

Referring to Figures 5a and 5b, there is illustrated a urine collection system according to an embodiment of the invention named the "Alternative catheter system (ACS)" by the Applicant. In Figures 5a and 5b, an embodiment of the second aspect of the invention is shown combined with an embodiment of the first aspect of the invention. The ACS is shown in situ in a male patient in Figures 5a and 5b.

Figure 5a shows a catheter having an abdominal stop 5 attached to the suprapubic section 1 and positioned against the abdomen, drainage holes 4 in the bladder section and a non-return cuff 6 attached to the bladder section 2 upstream of the drainage holes 4 (i.e. between the drainage holes and the abdominal stop) and inserted into the bladder.

Regarding the aspect of the invention, Figures 5a and 5b show the upper or suprapubic end of the catheter, beyond the abdominal stop 5, attached to an abdominal urine collection pouch 12 which will hold a volume typically up to 500 ml of urine. There is no valve between the catheter and the pouch. Urine can flow freely from the bladder into the pouch as the bladder fills or if the bladder muscle contracts. When the bladder muscle relaxes, urine can return into the bladder. This system allows the bladder to fill normally and if the bladder pressure rises to a threshold pressure, for example as a result of a contraction of the bladder (detrusor) muscle, urine enters the abdominal urine collection pouch and then returns to the bladder once the pressure falls below the threshold, for example as the bladder muscle relaxes. Thus a low pressure urinary reservoir is maintained.

The bladder and any urine collection pouch 12 are emptied by releasing the catheter valve 13 at the lower end of the catheter.

The catheter and the pouch provide a closed urine collection system. A Luer or similar fitting 14 may be included at the top of the pouch (e.g. as shown in Figure 5b) so that the pouch and catheter can be irrigated if necessary to clear any accumulated debris.

To flush the system, normal saline or other standard bladder washout solutions can be used to irrigate the bladder either through the Luer lock on the abdominal urine collection pouch or through the drainage funnel (not shown) at the upper end of the catheter. By releasing the catheter valve at the lower end of the catheter, the bladder can be flushed on a regular basis, the frequency depending on the amount of debris accumulating in the urine.

In one embodiment, as can be seen from the Figure 5b, the abdominal urine collection pouch 12 is attached to the user's abdomen by a waistband 15.

The manner of introduction of the catheter of the present invention will now be described. A transurethral flexible cystoscopy should initially be performed to exclude the presence of pathology in the urethra or bladder such as stricture, tumour or stones. The urethral end of the ACS catheter is inserted through the suprapubic cystotomy and when this becomes visible in the bladder, the end is held in a pair of endoscopic grabbing forceps. The cystoscope, grabbing forceps and catheter are withdrawn from the urethra.

Gentle traction is applied to the urethral end of the catheter until the non-return cuff on the bladder section of the catheter has been passed through the abdominal wall and entered the bladder. Gentle traction is then applied to the upper or suprapubic end until the non-return cuff engages with the bladder wall thus preventing further withdrawal of the catheter from the bladder. Having reached that point, the abdominal flange is adjusted by sliding it along the catheter until it rests gently against the abdominal wall. In that way the catheter is held in place between the flange on the surface of the body and the non-return cuff within the bladder.

With the catheter in situ, the suprapubic section of the catheter is trimmed and attached to the abdominal urine collection pouch following the instructions provided with the pouch. After trimming the urethral section of the catheter to a suitable length for the patient, a drainage funnel is fitted to the lower end of the catheter and a catheter valve introduced into this.

To change the catheter, one possibility is to detach the abdominal urine collection pouch from the suprapubic section of the catheter above the abdominal flange. The drainage funnel is detached from the urethral end of the catheter. A guidewire is passed through the catheter from the suprapubic end until it emerges from the end of the urethral section. Whilst the guidewire is held securely, the abdominal flange is removed from the suprapubic section by sliding it along to the upper end of the catheter following which the used catheter is removed from the urethral end. The new ACS is passed over the guide wire from the suprapubic end until it emerges from the urethra, the guide wire is removed and the same procedure as for the initial catheterisation is followed, securing the non-return valve in the bladder and adjusting the abdominal flange before attaching the abdominal urine collection pouch at the suprapubic end and drainage funnel at the urethral end. A standard catheter valve is introduced into the drainage funnel.

Alternatively, the abdominal urine collection pouch may be detached from the suprapubic section of the catheter above the abdominal flange. Holding the catheter firmly between the abdominal wall and the abdominal flange, the flange is removed by sliding it off the suprapubic end of the catheter and a small connecting rod is inserted into the lumen at the end of the suprapubic section. The lumen at the urethral end of the new catheter is then inserted over the connecting rod and when firmly attached, gentle traction is applied to the urethral end of the used catheter to pull it together with the new catheter through the abdominal wall, bladder and urethra until it emerges from the lower or urethral end. The used catheter and the connecting rod are removed from the new catheter and the same procedure is followed to introduce the non-return cuff into the bladder, adjust the abdominal flange and attach the abdominal urine collection pouch and drainage funnel into the suprapubic and urethral ends of the new catheter respectively. A standard catheter valve is introduced into the drainage funnel.

The bladder and the urine collection pouch are emptied by releasing the catheter valve 13 at the lower end of the ACS. To flush the urine collection pouch and bladder, irrigation can be performed through the Luer or similar fitting 14 on the abdominal urine collection pouch using any standard bladder instillation or washout solution and by releasing the catheter valve 13.

The ACS provides a versatile means of draining the bladder. As an alternative to the abdominal urine collection pouch, a drainage funnel can be attached to the upper or suprapubic section of the catheter. With a drainage funnel at both ends, the bladder can be drained or flushed with a standard bladder irrigation fluid from either or both ends or using a catheter valve or a urine collection bag at one end and a spigot at the other. Bladder irrigation using standard bladder washout and other solutions can be performed from either end.

### References

1. Saint S, Elmore JG, Sullivan SD, Emerson SS, Koepsell TD. The efficacy of silver alloy-coated urinary catheters in preventing urinary tract infection: a meta-analysis. Am J Med 1998: 105: 236-241).
2. Maki DG, Tambyah PA. Engineering Out the Risk for infection with Urinary Catheters. Emerging Infectious Diseases 2001: 7(2):342-347.
3. Saint S. Clinical and economic consequences of nosocomial catheter-related bacteriuria. Am J Infect Control 2000: 28(1):68-73.
4. Rosenthal VD. Device-associated nosocomial infections in limited-resources countries: findings of the International Nosocomial Infection Control Consortium (INICC). Am J Infect Control 2008: 36(10):S171-12.
5. Foley FEB. A Self-Retaining Bag Catheter. J Urol 1937: 38:140-143.
6. Warren, JW Catheter-associated urinary tract infections Infect Dis North Am 11 (3): 609-622 1997.
7. Kohler-Ockmore J. and Feneley RCL. Long-term catheterisation of the bladder: prevalence and morbidity. British Journal of Urology. 77: 347-351 1996
8. Hellstrom L, Ekeland P, Milsom I, and Mellstrom D. Prevalence of incontinence: use of aids. Age and Ageing. 19: 383-389 1990.
9. Kunin, CM. Can we build a better urinary catheter? N Eng J Med 319 (6):365-366, 1988.

## Claims

1. A urine collection system comprising:
a suprapubic urethral catheter comprising a hollow tube having sequentially, a suprapubic section (1), a bladder section (2) and a urethral section (3), wherein, the bladder section (2) comprises one or more drainage holes (4) in the wall of the tube ; and
a urine collection container (12) connected to the suprapubic section (1),
wherein:
the suprapubic section (1) provides a flowpath between the bladder section (2) and the urine collection container (12), so that when the bladder section (2) is located within a bladder of a patient, urine flows along the flowpath from the bladder to the urine collection container (12) when the pressure in the bladder rises above a threshold pressure, and then returns along the flowpath from the urine collection container (12) to the bladder when the pressure falls below the threshold pressure, thus providing a low pressure reservoir,
**characterised in that**:
there is an abdominal stop (5) attached to the suprapubic section (1).

2. A urine collection system according to claim 1, including a waistband (15) for attaching the urine collection container (12) to a patient.

3. A urine collection system according to claim 1 or claim 2, wherein the urine collection container (12) is a pouch.

4. A urine collection system according to any one of claims 1 to 3, wherein the urine collection container (12) includes a Luer fitting (14).

5. A urine collection system according to any one of claims 1 to 3, wherein the suprapubic urethral catheter has a non-return cuff (6) attached to the bladder section (2) between the one or more drainage holes (4) and the abdominal stop (5); and wherein, the non-return cuff (6) has a projecting portion (10) projecting outwardly from the tube which is deformable towards the tube and which is adapted to resist deformation away from the tube.

6. A urine collection system according to claim 5, wherein the projecting portion (10) of the cuff (6) comprises a wing (10) inclined away from the tube, wherein the end of the wing (10) furthest from the tube is nearest the abdominal stop (5).

7. A urine collection system according to claim 6, wherein the projecting portion (10) of the cuff comprises two inclined wings (10).

8. A urine collection system according to claim 7, wherein the non-return cuff (6) includes a channel (8) for receiving the tube of the catheter, and the two inclined wings (10) are positioned on opposite sides of the channel (8).

9. A urine collection system according to claim 8, wherein the two inclined wings (10) are joined by a resiliently deformable element (11) and the wings (10) are biased apart from each other by the resiliently deformable element (11).

10. A urine collection system according to claim 9, wherein at the end of the wing (10) furthest from the abdominal stop (5), the non-return cuff (6) has a sleeve (9) formed around the channel (8) and attached to the tube.

11. A urine collection system according to claim 10, wherein at the end of the sleeve (9) furthest from the abdominal stop (5), the walls of the sleeve taper towards the tube.

12. A urine collection system according to any one of claims 7 to 11, wherein each wing (10) is shaped as a distorted elongated polygon, wherein the polygon is elongated along the length of the wing (10), and the widest part of the polygon is located towards the end of the wings (10) being furthest from the abdominal stop (5).

13. A urine collection system according to any one of claims 5 to 12, wherein there is a plurality of said drainage holes (4) located along the bladder section (2) .

14. A urine collection system according to any one of claims 5 to 13 wherein there is at least one irrigation hole (7) in the wall of the tube positioned in the bladder section (2) within the projecting portion (10) of the non-return cuff (6).

15. A urine collection system according to any one of claims 5 to 14, wherein the abdominal stop (5) has a channel (18) for receiving and gripping the suprapubic section (1) of the catheter.

16. A urine collection system according to claim 15, wherein the position of the abdominal stop (5) along the suprapubic section (1) is adjustable.

17. A urine collection system according to any one of claims 5 to 16, wherein there is a radio-opaque or ultrasonic marker (17) at the junction between the bladder section (2) and the urethral section (3).

18. A urine collection system according to any one of claims 5 to 17, wherein the urethral section (3) is connected to a drainage funnel or connector (13).

19. A urine collection system according to claim 18, wherein the drainage funnel or connector (13) includes a valve (13) for opening or closing the flowpath through the urethral section (3).

20. A kit of parts for assembling a urine collection system, the kit comprising:
a suprapubic urethral catheter comprising a hollow tube having sequentially, a suprapubic section (1), a bladder section (2) and a urethral section (3), wherein the bladder section (2) comprises one or more drainage holes (4) in the wall of the tube; and
an abdominal urine collection container (12) connectable to the suprapubic section (1),
wherein:
the suprapubic section (1) is arranged to provide a flowpath between the bladder section (2) and the abdominal urine collection container (12), so that when the bladder section (2) is located within a bladder of a patient, urine flows along the flowpath from the bladder to the abdominal urine collection container (12) when the pressure in the bladder rises above a threshold pressure, and then returns along the flowpath from the abdominal urine collection container (12) to the bladder when the pressure falls below the threshold pressure, thus providing a low pressure reservoir,
**characterised in that**:
the kit of parts further comprises an abdominal stop (5) attachable to the suprapubic section (1).

21. A kit of parts according to claim 20, the kit further comprising:
a non-return cuff (6) which is attachable to the bladder section (2) between the one or more drainage holes (4) and the abdominal stop (5), wherein the non-return cuff (6) has a projecting portion (10) projecting outwardly from the tube which is deformable towards the tube and which is adapted to resist deformation away from the tube.

22. A kit of parts according to claim 20 or claim 21, the kit further comprising a waistband (15).

## Patentansprüche

1. Urinsammelsystem, das Folgendes umfasst:
einen suprapubischen Harnröhrenkatheter, der einen hohlen Schlauch umfasst, der nacheinander einen suprapubischen Abschnitt (1), einen Blasenabschnitt (2) und einen Harnröhrenabschnitt (3) umfasst,
wobei der Blasenabschnitt (2) ein oder mehrere Abflusslöcher (4) in der Wand des Schlauchs umfasst; und
einen Urinsammelbehälter (12), der mit dem suprapubischen Abschnitt (1) verbunden ist,
wobei:
der suprapubische Abschnitt (1) einen Strömungspfad zwischen dem Blasenabschnitt (2) und dem Urinsammelbehälter (12) bereitstellt, so dass, wenn sich der Blasenabschnitt (2) innerhalb einer Blase eines Patienten befindet, Urin entlang des Strömungspfads von der Blase in den Urinsammelbehälter (12) strömt,
wenn der Druck in der Blase über einen Schwellendruck ansteigt, und dann entlang des Strömungspfads von dem Urinsammelbehälter (12) in Richtung der Blase zurückströmt, wenn der Druck unter den Schwellendruck fällt, wodurch ein Niederdruckreservoir bereitgestellt wird,
**dadurch gekennzeichnet, dass**:
es einen Abdominalstopp (5) gibt, der mit dem suprapubischen Abschnitt (1) verbunden ist.

2. Urinsammelsystem nach Anspruch 1, das einen Bauchgurt (15) zum Befestigen des Urinsammelbehälters (12) an einem Patienten umfasst.

3. Urinsammelsystem nach Anspruch 1 oder 2, wobei der Urinsammelbehälter (12) ein Beutel ist.

4. Urinsammelsystem nach einem der Ansprüche 1 bis 3, wobei der Urinsammelbehälter (12) einen Luer-Anschluss (14) umfasst.

5. Urinsammelsystem nach einem der Ansprüche 1 bis 3, wobei der suprapubische Harnröhrenkatheter eine Rückschlagklemme (6) aufweist, zwischen dem einen oder mehreren Abflusslöchern (4) und dem Abdominalstopp (5) mit dem Blasenabschnitt (2) verbunden ist; und wobei die Rückschlagklemme (6) einen Vorsprungabschnitt (10) aufweist, der von dem Schlauch nach außen hervorsteht, welcher in Richtung des Schlauchs verformbar ist, und der ausgelegt ist, einer Verformung in eine dem Schlauch abgewandte Richtung zu widerstehen.

6. Urinsammelsystem nach Anspruch 5, wobei der Vorsprungabschnitt (10) der Klemme (6) einen Flügel (10) umfasst, der von dem Schlauch weggeneigt ist,
wobei sich das Ende des Flügels (10), das am weitesten von dem Schlauch entfernt ist, am nächsten zu dem Abdominalstopp (5) befindet.

7. Urinsammelsystem nach Anspruch 6, wobei der Vorsprungabschnitt (10) der Klemme zwei geneigte Flügel (10) umfasst.

8. Urinsammelsystem nach Anspruch 7, wobei die Rückschlagklemme (6) einen Kanal (8) zum Empfangen des Schlauchs des Katheters umfasst, und die zwei geneigten Flügel (10) an gegenüberliegenden Seiten des Kanals (8) positioniert sind.

9. Urinsammelsystem nach Anspruch 8, wobei die zwei geneigten Flügel (10) über ein elastisch verformbares Element (11) verbunden sind, und die Flügel (10) von dem elastisch verformbaren Element (11) voneinander beabstandet werden.

10. Urinsammelsystem nach Anspruch 9, wobei die Rückschlagklemme (6) am Ende des Flügels (10), das sich am weitesten von dem Abdominalstopp (5) entfernt befindet, eine Hülle (9) aufweist, die rund um den Kanal (8) ausgebildet und an dem Schlauch befestigt ist.

11. Urinsammelsystem nach Anspruch 10, wobei sich die Wände der Hülle am Ende der Hülle (9), das am weitesten von dem Abdominalstopp (5) entfernt ist, in Richtung des Schlauchs hin verjüngen.

12. Urinsammelsystem nach einem der Ansprüche 7 bis 11, wobei jeder Flügel (10) als verdrehtes längliches Vieleck geformt ist, wobei die Längsseite des Vielecks entlang der Länge des Flügels (10) verläuft, und sich der breiteste Teil des Vielecks in Richtung des Endes der Flügel (10) befindet, das am weitesten von dem Abdominalstopp (5) entfernt ist.

13. Urinsammelsystem nach einem der Ansprüche 5 bis 12, wobei es eine Vielzahl der Abflusslöcher (4) gibt, die sich entlang des Blasenabschnitts (2) befinden.

14. Urinsammelsystem nach einem der Ansprüche 5 bis 13, wobei es zumindest ein Spülloch (7) in der Wand des Schlauchs gibt, das in dem Blasenabschnitt (2) innerhalb des Vorsprungabschnitts (10) der Rückschlagklemme (6) positioniert ist.

15. Urinsammelsystem nach einem der Ansprüche 5 bis 14, wobei der Abdominalstopp (5) einen Kanal (18) zum Empfangen und Greifen des suprapubischen Abschnitts (1) des Katheters aufweist.

16. Urinsammelsystem nach Anspruch 15, wobei die Position des Abdominalstopps (5) entlang des suprapubischen Abschnitts (1) einstellbar ist.

17. Urinsammelsystem nach einem der Ansprüche 5 bis 16, wobei es einen radioopaken oder Ultraschallmarker (17) an dem Übergang zwischen dem Blasenabschnitt (2) und dem Harnröhrenabschnitt (3) gibt.

18. Urinsammelsystem nach einem der Ansprüche 5 bis 17, wobei der Harnröhrenabschnitt (3) mit einem Abflusstrichter oder einem Anschlussstück (13) verbunden ist.

19. Urinsammelsystem nach Anspruch 18, wobei der Abflusstrichter oder das Anschlussstück (13) ein Ventil (13) zum Öffnen oder Schließen des Strömungspfads durch den Harnröhrenabschnitt (3) umfasst.

20. Set aus Teilen zum Zusammenstellen eines Urinsammelsystems, wobei das Set Folgendes umfasst:
einen suprapubischen Harnröhrenkatheter, der einen hohlen Schlauch umfasst, der nacheinander einen suprapubischen Abschnitt (1), einen Blasenabschnitt (2) und einen Harnröhrenabschnitt (3) umfasst, wobei der Blasenabschnitt (2) ein oder mehrere Abflusslöcher (4) in der Wand des Schlauchs umfasst; und
einen abdominalen Urinsammelbehälter (12), der mit dem suprapubischen Abschnitt (1) verbunden werden kann,
wobei:
der suprapubische Abschnitt (1) angeordnet ist, um einen Strömungspfad zwischen dem Blasenabschnitt (2) und dem abdominalen Urinsammelbehälter (12) bereitzustellen, so dass, wenn sich der Blasenabschnitt (2) innerhalb einer Blase eines Patienten befindet, Urin entlang des Strömungspfads von der Blase in den abdominalen Urinsammelbehälter (12) strömt, wenn der Druck in der Blase über einen Schwellendruck ansteigt, und dann entlang des Strömungspfads von dem abdominalen Urinsammelbehälter (12) in Richtung Blase zurückströmt, wenn der Druck unter den Schwellendruck fällt, wodurch ein Niederdruckreservoir bereitgestellt wird,
**dadurch gekennzeichnet, dass**:
das Set aus Teilen ferner einen Abdominalstopp (5) umfasst, der an dem suprapubischen Abschnitt (1) befestigt werden kann.

21. Set aus Teilen nach Anspruch 20, wobei das Set ferner Folgendes umfasst:
eine Rückschlagklemme (6), die zwischen dem einen oder mehreren Abflusslöchern (4) und dem Abdominalstopp (5) an dem Blasenabschnitt (2) befestigt werden kann, wobei die Rückschlagklemme (6) einen Vorsprungabschnitt (10) umfasst, der von dem Schlauch nach außen hervorsteht, der in Richtung des Schlauchs verformbar ist, und der ausgelegt ist, einer Verformung in eine dem Schlauch abgewandte Richtung zu widerstehen.

22. Set aus Teilen nach Anspruch 20 oder Anspruch 21, wobei das Set ferner einen Bauchgurt (15) umfasst.

## Revendications

1. Système de collecte d'urine comprenant :
un cathéter urétral sus-pubien comprenant un tube creux ayant, en séquence, une section sus-pubienne (1), une section de vessie (2) et une section urétrale (3),
dans lequel, la section de vessie (2) comprend un ou plusieurs trous de drainage (4) dans la paroi du tube ; et
un récipient de collecte d'urine (12) raccordé à la section sus-pubienne (1),
dans lequel :
la section sus-pubienne (1) fournit une trajectoire d'écoulement entre la section de vessie (2) et le récipient de collecte d'urine (12), de sorte que lorsque la section de vessie (2) est positionnée à l'intérieur d'une vessie d'un patient, l'urine s'écoule le long de la trajectoire d'écoulement de la vessie jusqu'au récipient de collecte d'urine (12) lorsque la pression dans la vessie augmente au-dessus d'une pression de seuil, et revient ensuite le long de la trajectoire d'écoulement du récipient de collecte d'urine (12) à la vessie lorsque la pression chute au-dessous d'une pression de seuil, fournissant ainsi un réservoir basse pression,
**caractérisé en ce que** :
il y a une butée abdominale (5) fixée à la section sus-pubienne (1).

2. Système de collecte d'urine selon la revendication 1, comprenant une ceinture (15) pour fixer le récipient de collecte d'urine (12) sur un patient.

3. Système de collecte d'urine selon la revendication 1 ou la revendication 2, dans lequel le récipient de collecte d'urine (12) est une poche.

4. Système de collecte d'urine selon l'une quelconque des revendications 1 à 3, dans lequel le récipient de collecte d'urine (12) comprend un raccord Luer (14).

5. Système de collecte d'urine selon l'une quelconque des revendications 1 à 3, dans lequel le cathéter urétral sus-pubien a une manchette de non retour (6) fixée à la section de vessie (2) entre les un ou plusieurs trous de drainage (4) et la butée abdominale (5) ; et dans lequel, la manchette de non retour (6) a une partie en saillie (10) faisant saillie vers l'extérieur du tube qui est déformable vers le tube et qui est adaptée pour résister à la déformation à distance du tube.

6. Système de collecte d'urine selon la revendication 5, dans lequel la partie en saillie (10) de la manchette (6) comprend une aile (10) inclinée à distance du tube,
dans lequel l'extrémité de l'aile (10) la plus éloignée du tube est la plus proche de la butée abdominale (5) .

7. Système de collecte d'urine selon la revendication 6, dans lequel la partie en saillie (10) de la manchette comprend deux ailes inclinées (10).

8. Système de collecte d'urine selon la revendication 7, dans lequel la manchette de non retour (6) comprend un canal (8) pour recevoir le tube du cathéter, et les deux ailes inclinées (10) sont positionnées sur les côtés opposés du canal (8).

9. Système de collecte d'urine selon la revendication 8, dans lequel les deux ailes inclinées (10) sont assemblées par un élément déformable de manière résiliente (10) et les ailes (10) sont sollicitées à distance l'une de l'autre par l'élément déformable de manière résiliente (11) .

10. Système de collecte d'urine selon la revendication 9, dans lequel à l'extrémité de l'aile (10) la plus éloignée de la butée abdominale (5), la manchette de non retour (6) a un manchon (9) formée autour du canal (8) et fixé au tube.

11. Système de collecte d'urine selon la revendication 10, dans lequel à l'extrémité du manchon (9) la plus éloignée de la butée abdominale (5), les parois du manchon se rétrécissent progressivement vers le tube.

12. Système de collecte d'urine selon l'une quelconque des revendications 7 à 11, dans lequel chaque aile (10) est formée comme un polygone allongé déformé, dans lequel le polygone est allongé le long de la longueur de l'aile (10), et la partie la plus large du polygone est positionnée vers l'extrémité des ailes (10) qui est la plus éloignée de la butée abdominale (5).

13. Système de collecte d'urine selon l'une quelconque des revendications 5 à 12, dans lequel il y a une pluralité desdits trous de drainage (4) positionnés le long de la section de vessie (2).

14. Système de collecte d'urine selon l'une quelconque des revendications 5 à 13, dans lequel il y a au moins un trou d'irrigation (7) dans la paroi du tube positionné dans la section de vessie (2) à l'intérieur de la partie en saillie (10) de la manchette de non retour (6).

15. Système de collecte d'urine selon l'une quelconque des revendications 5 à 14, dans lequel la butée abdominale (5) a un canal (18) pour recevoir et saisir la section sus-pubienne (1) du cathéter.

16. Système de collecte d'urine selon la revendication 15, dans lequel la position de la butée abdominale (5) le long de la section sus-pubienne (1) est ajustable.

17. Système de collecte d'urine selon l'une quelconque des revendications 5 à 16, dans lequel il y a un marqueur radio-opaque ou ultrasonore (17) au niveau de la jonction entre la section de vessie (2) et la section urétrale (3).

18. Système de collecte d'urine selon l'une quelconque des revendications 5 à 17, dans lequel la section urétrale (3) est raccordée à un entonnoir ou connecteur de drainage (13) .

19. Système de collecte d'urine selon la revendication 18, dans lequel l'entonnoir ou connecteur de drainage (13) comprend une valve (13) pour ouvrir ou fermer la trajectoire d'écoulement à travers la section urétrale (3).

20. Kit de pièces pour assembler un système de collecte d'urine, le kit comprenant :
un cathéter urétral sus-pubien comprenant un tube creux ayant, en séquence, une section sus-pubienne (1), une section de vessie (2) et une section urétrale (3), dans lequel la section de vessie (2) comprend un ou plusieurs trous de drainage (4) dans la paroi du tube ; et
un récipient de collecte d'urine abdominal (12) pouvant être raccordé à la section sus-pubienne (1),
dans lequel :
la section sus-pubienne (1) est agencée pour fournir une trajectoire d'écoulement entre la section de vessie (2) et le récipient de collecte d'urine abdominal (12), de sorte que lorsque la section de vessie (2) est positionnée à l'intérieur de la vessie d'un patient, l'urine s'écoule le long de la trajectoire d'écoulement de la vessie au récipient de collecte d'urine abdominal (12) lorsque la pression dans la vessie augmente au-dessus d'une pression de seuil, et revient ensuite le long de la trajectoire d'écoulement du récipient de collecte d'urine abdominal (12) à la vessie lorsque la pression chute au-dessous d'une pression de seuil, fournissant ainsi un réservoir basse pression,
**caractérisé en ce que** :
le kit de pièces comprend en outre une butée abdominale (5) pouvant être fixée à la section sus-pubienne (1) .

21. Kit de pièces selon la revendication 20, le kit comprenant en outre :
une manchette de non retour (6) qui peut être fixée à la section de vessie (2) entre les un ou plusieurs trous de drainage (4) et la butée abdominale (5), dans lequel la manchette de non retour (6) a une partie en saillie (10) faisant saillie vers l'extérieur à partir du tube qui est déformable vers le tube et qui est adaptée pour résister à la déformation à distance du tube.

22. Kit de pièces selon la revendication 20 ou la revendication 21, le kit comprenant en outre une ceinture (15) .
